# EUROPÄISCHE PATENTSCHRIFT

(11) **EP 4 082 591 B1**
(45) Veröffentlichungstag und Bekanntmachung des Hinweises auf die Patenterteilung: **08.04.2026**
(21) Anmeldenummer: 22169899.6
(22) Anmeldetag: 26.04.2022
(51) Int. Cl.: A61M 16/00, A61B 5/00

(54) **BEATMUNGSSYSTEM MIT WENIGSTENS EINEM BEATMUNGSGERÄT UND WENIGSTENS EINEM DIAGNOSEGERÄT**
VENTILATION SYSTEM WITH AT LEAST ONE VENTILATOR AND AT LEAST ONE DIAGNOSTIC DEVICE
SYSTÈME RESPIRATOIRE POURVU D'AU MOINS UN APPAREIL RESPIRATOIRE ET D'AU MOINS UN APPAREIL DE DIAGNOSTIC

(30) Priorität: 30.04.2021 DE 102021111291
(43) Veröffentlichungstag der Anmeldung: 02.11.2022
(73) Patentinhaber: Löwenstein Medical Technology S.A., 2557 Luxembourg (LU)
(72) Erfinder: Schwaibold, Matthias, 76228 Karlsruhe (DE); Verhoeven, Jan, 76275 Ettlingen (DE); Furthmueller, Jochen, 76287 Rheinstetten (DE)
(74) Vertreter: Löwenstein Medical IP

(56) Entgegenhaltungen:
- US-A1- 2011 209 704
- US-A1- 2014 073 889
- US-A1- 2018 221 608

## Beschreibung

Die vorliegende Erfindung betrifft ein Beatmungssystem mit wenigstens einem Beatmungsgerät und mit wenigstens einem Diagnosegerät. Das Beatmungsgerät umfasst wenigstens eine Beatmungseinrichtung zur Erzeugung einer Atemgasströmung für eine Beatmung und wenigstens eine Erfassungseinrichtung zur Erfassung wenigstens eines für die Atemgasströmung charakteristischen Beatmungssignals über die Zeit. Das Diagnosegerät umfasst wenigstens eine Sensoreinrichtung zur Erfassung wenigstens eines Diagnosesignals über die Zeit. Solche Beatmungssysteme werden üblicherweise zur Therapiekontrolle eingesetzt. In der Regel nutzt der Patient über einen längeren Zeitraum das Beatmungsgerät, beispielsweise im Rahmen einer Schlaf therapie. Für die Therapiekontrolle wird dann zusätzlich das Diagnosegerät zur Verfügung gestellt, um den Zustand des Patienten unmittelbar während der Beatmung zu erfassen. So können die Qualität und Wirksamkeit der Beatmung überwacht und bei Bedarf die Beatmungseinstellungen gezielt an die Bedürfnisse des Patienten angepasst werden.

Im Rahmen der Therapiekontrolle werden somit neben den ohnehin mit dem Beatmungsgerät erfassten Parameter zusätzlich oft noch viele weitere Größen von den Sensoren des Diagnosegeräts gemessen und aufgezeichnet. Für eine aussagekräftige und verlässliche Therapiekontrolle ist es besonders entscheidend, die Signale vom Beatmungsgerät mit den Signalen vom Diagnosegerät miteinander in Bezug zu setzen und zu vergleichen. Dabei ist es unerlässlich, dass die Signale aus dem Beatmungsgerät mit denen aus dem Diagnosegerät zeitlich übereinstimmen bzw. synchron sind.

Eine solche Synchronisation der im Rahmen der Therapiekontrolle erfassten Daten ist jedoch mit einem erheblichen Aufwand verbunden. Daher werden solche Therapiekontrollen häufig in einem Schlaflabor durchgeführt, welches ein umfangreiches Netzwerk aufweist, in dem die Sensoren des Beatmungsgerätes und des Diagnosegerätes miteinander verschaltet sind. Dadurch können alle erfassten Daten gleichzeitig zentral gespeichert werden. Allerdings stellt der Besuch in einem Schlaflabor oft einen anstrengenden und unangenehmen Aufwand für den Patienten dar. Zudem spiegeln die im Schlaflabor gewonnenen Daten aufgrund der ungewohnten Schlafsituation mitunter nicht die tatsächliche Beatmungssituation des Patienten wider.

Daher erfolgt die Therapiekontrolle bei einer Heimbeatmung auch immer häufiger dadurch, dass dem Patienten ein Diagnosegerät für eine oder mehrere Nächte überlassen wird. Anschließend müssen sowohl die Daten aus dem Diagnosegerät als auch aus dem Beatmungsgerät ausgelesen und in aufwendiger Arbeit durch Fachpersonal synchronisiert werden.

Um die Synchronisation zu vereinfachen, könnten die internen Uhren von Diagnosegerät und Beatmungsgerät genutzt werden. Allerdings hat sich dies für eine automatisierte Synchronisation als unzureichend erwiesen, da die internen Uhren von Beatmungsgerät und Diagnosegerät in der Regel nicht ausreichend synchron laufen oder sich während der Aufzeichnung ein störender Gangunterschied ergibt.

US 2018/0221608 A1 beschreibt ein Verfahren zum Betreiben eines Beatmungsgeräts, bei dem ein Druck- mit einem Flusssignal synchronisiert wird.

US 2011/0209704 A1 beschreibt ein Verfahren zur Bestimmung einer Übertragungsverzögerung in Verbindung mit einem verteilten Sensor in einem Beatmungssystem. Dabei können Druck- und Flussdaten miteinander synchronisiert werden.

US 2014/0073889 A1 beschreibt ein Verfahren zur Bestimmung einer Flüssigkeitsempfindlichkeit eines beatmeten Patienten, bei dem ein Druck-, Volumen- oder Flusssignal mit einem physiologischen Signal wie etwa einem PPG-Signal synchronisiert werden kann.

Es ist daher die Aufgabe der vorliegenden Erfindung, ein Beatmungssystem zur Verfügung zu stellen, welches eine verbesserte Synchronisation des Beatmungssignals mit dem Diagnosesignal im Rahmen einer Therapiekontrolle ermöglicht. Insbesondere soll die Synchronisation derart automatisiert möglich sein, dass der Patient für die Therapiekontrolle zu Hause bleiben kann bzw. nicht in Schlaflabor muss und auf eine aufwendige Auswertung durch Fachpersonal verzichtet werden kann. Zugleich soll das Beatmungssystem vorzugsweise technisch bzw. konstruktiv unaufwendig sein und auch durch ungeübte Benutzer sicher bedienbar sein.

Diese Aufgabe wird gelöst mit einem Beatmungssystem des Anspruchs 1 Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Beatmungssystem umfasst wenigstens ein Beatmungsgerät und wenigstens ein Diagnosegerät. Das Beatmungsgerät umfasst wenigstens eine Beatmungseinrichtung zur Erzeugung einer Atemgasströmung für eine Beatmung. Das Beatmungsgerät umfasst wenigstens eine Erfassungseinrichtung zur Erfassung wenigstens eines Beatmungssignals über die Zeit, welches charakteristisch für die Atemgasströmung ist. Das Diagnosegerät umfasst wenigstens eine Sensoreinrichtung zur Erfassung wenigstens eines Diagnosesignals über die Zeit. Dabei umfasst das Beatmungssystem wenigstens eine Synchronisationseinheit. Die Synchronisationseinheit ist mit dem Beatmungsgerät und insbesondere wenigstens mit dessen Erfassungseinrichtung wirkverbunden. Die Synchronisationseinheit ist mit dem Diagnosegerät und insbesondere wenigstens mit dessen Sensoreinrichtung wirkverbunden. Die Synchronisationseinheit ist dazu geeignet und ausgebildet, wenigstens einen zeitlichen Verlauf des Beatmungssignals und wenigstens einen zeitlichen Verlauf des Diagnosesignals jeweils auf wenigstens eine durch dasselbe Ereignis verursachte Signaländerung zu untersuchen. Mit anderen Worten, die Erfassungseinrichtung ist insbesondere dazu geeignet und ausgebildet, eine Signaländerung im zeitlichen Verlauf des Beatmungssignals und eine Signaländerung im zeitlichen Verlauf des Diagnosesignals zu identifizieren, wobei die Signaländerungen durch dasselbe Ereignis verursacht sind bzw. verursacht wurden. Dabei ist die Synchronisationseinheit dazu geeignet und ausgebildet, den Verlauf des Beatmungssignals und den Verlauf des Diagnosesignals (wenigstens näherungsweise) in eine zeitliche Übereinstimmung (Synchronität) zu bringen bzw. zu synchronisieren, sodass das Ereignis im Verlauf des Beatmungssignals und im Verlauf des Diagnosesignals (wenigstens näherungsweise) zeitgleich auftritt.

Das erfindungsgemäße Beatmungssystem bietet viele Vorteile. Einen erheblichen Vorteil bietet die Synchronisationseinheit mit ihrer Fähigkeit, Signaländerungen im Beatmungssignal und Diagnosesignal zu finden, welche durch zeitgleiche Ereignisse ausgelöst wurden. Dadurch kann die Synchronisation automatisiert erfolgen, sodass die Therapiekontrolle bequem auch zu Hause erfolgen kann und insgesamt für die Auswertung Aufwand und Kosten eingespart werden können. Zudem bietet die Erfindung eine technisch bzw. konstruktiv unaufwendige und zugleich einfach zu bedienende Möglichkeit der automatisierten Synchronisation. So kann auch der Patient die Therapiekontrolle mit einem zur Verfügung gestellten Diagnosegerät bei sich zu Hause alleine durchführen. Des Weiteren bietet die Erfindung eine besonders genaue bzw. reproduzierbare Synchronisation. Beispielsweise kann damit sogar eine Beurteilung des Triggerverhaltens des Beatmungsgerätes erfolgen.

Nach der vollautomatischen Synchronisierung kann in einer bevorzugten Ausführung auch noch eine manuelle Feinjustierung vorgenommen werden. Dies ist insbesondere dann hilfreich, wenn die zur Synchronisierung verwendeten Signale zeitlich nicht hoch genug aufgelöst sind oder aufgrund von technischen Einflüssen, z. B. Filterung oder Verstärkung, zeitlich leicht zueinander versetzt sind. In diesem Fall kann der Auswerter nach der automatischen Synchronisation eine Korrektur vornehmen anhand seiner visuellen Beurteilung. Die Korrektur kann typischerweise von bis zu 10 Sekunden in Schritten von bis zu 0,5 Sekunden vorgenommen werden. Beispielsweise kann die Korrektur von 5 Sekunden in Schritten von 0,1 Sekunden vorgenommen werden. Bevorzugt kann die Korrektur von 1 Sekunde in Schritten von 0,05 Sekunden vorgenommen werden anhand seiner visuellen Beurteilung. Damit wird immer noch viel Zeit gespart, um den ungefähren Versatz der Signale zu kompensieren, und dennoch kann die Präzision einer visuell bestimmten Justierung erreicht werden.

Vorzugsweise ist die Synchronisationseinheit dazu geeignet und ausgebildet, durch die Signaländerungen, welche durch dasselbe Ereignis verursacht sind, wenigstens einen eindeutigen Zeitpunkt im zeitlichen Verlauf des Beatmungssignals und im zeitlichen Verlauf des Diagnosesignals zu erkennen und die Signalverläufe auf diesen Zeitpunkt zu synchronisieren. Insbesondere dient der Zeitpunkt des Ereignisses als Bezugspunkt für Synchronisation. Insbesondere werden für die Synchronisation die Zeitachsen in den zeitlichen Verläufen des Beatmungssignals und des Diagnosesignals jeweils so in Überstimmung gebracht, dass die auf demselben Ereignis beruhenden Signaländerungen an gleicher Position auf der Zeitachse liegen. Insbesondere ist die Synchronisationseinheit dazu geeignet und ausgebildet, ohne synchronisierte Uhren die Signalverläufe miteinander zu synchronisieren und vorzugsweise auch zu analysieren.

Beispielsweise ist das Ereignis ein Auftreten einer Atemstörung des Patienten. Beispielsweise verursacht die Atemstörung eine Druckänderung und/oder Flussänderung im Signalverlauf des Beatmungsgerätes. Das Diagnosegerät erfasst beispielsweise über einen am Thorax befestigten Sensor die Atembewegungen, sodass die Atemstörung auch eine signifikante Signaländerung in diesem Signal verursacht. Die beiden Signaländerungen beruhen also auf demselben Ereignis, nämlich der Atemstörung. Da die Atemstörung ursächlich für beide Signaländerungen ist, können die Signalverläufe mit dem Zeitpunkt der Atemstörung als Bezugspunkt verlässlich synchronisiert werden. Dazu wird wenigstens eine Zeitachse so verschoben, dass die durch die Atemstörung verursachten Signaländerungen zeitlich übereinstimmen.

In einer vorteilhaften Weiterbildung ist die Synchronisationseinheit dazu geeignet und ausgebildet, ein Maß für eine Ähnlichkeit der Signaländerungen im zeitlichen Verlauf des Beatmungssignals und im zeitlichen Verlauf des Diagnosesignals zu ermitteln. Vorzugsweise bestimmt die Synchronisationseinheit in Abhängigkeit der Ähnlichkeit, ob die Signaländerungen auf demselben Ereignis beruhen oder nicht. Das ermöglicht ein unaufwendiges und zugleich zuverlässiges Auffinden von Signaländerungen, welche dann als Bezugspunkt für die Synchronisation dienen.

Das Beatmungssignal und/oder das Diagnosesignal umfassen insbesondere jeweils wenigstens eine über die Zeit erfasste Messgröße oder werden durch eine solche gebildet. Die Signaländerung umfasst dann insbesondere wenigstens eine charakteristische Änderung der Messgröße oder entspricht einer solchen. Zusätzlich oder alternativ kann das Beatmungssignal und/oder das Diagnosesignal jeweils auch wenigstens eine dem Signalverlauf hinzugefügte bzw. aufgelagerte und eindeutig als Signaländerung identifizierbare Information umfassen, welche insbesondere unabhängig von der Messgröße ist. Eine solche Information ist zum Beispiel das durch eine Benutzereingabe erzeugte Synchronisationssignal (nachfolgend beschrieben) oder der (nachfolgend beschriebene) Zeitstempel, welcher durch das Eintreffen und/oder Absenden eines Datenpakets generiert wird. Solche Informationen ermöglichen ebenfalls eine zuverlässige Synchronisation und lösen die erfindungsgemäße Aufgabe auch besonders vorteilhaft.

Das Maß für die Ähnlichkeit ist insbesondere eine Dauer und/oder eine Intensität und/oder eine Symmetrieeigenschaft. Das Maß für die Ähnlichkeit kann auch Veränderungsrate und/oder eine andere geeignete geometrische Struktur der Signaländerung betreffen. Das Maß für die Ähnlichkeit kann auch eine Häufigkeit und/oder Regelmäßigkeit der Signaländerungen in den jeweiligen zeitlichen Verläufen betreffen.

Erfindungsgemäß ist die Synchronisationseinheit dazu geeignet und ausgebildet, den zeitlichen Verlauf des Beatmungssignals und den zeitlichen Verlauf des Diagnosesignals jeweils auf eine Mehrzahl von Signaländerungen zu untersuchen. Die Signaländerungen beruhen jeweils paarweise (also jeweils eine Signaländerung des Beatmungssignals und jeweils eine Signaländerung des Diagnosesignals) auf demselben Ereignis. Insbesondere werden wenigstens zwei und vorzugsweise eine Mehrzahl von paarweisen Signaländerungen für die Synchronisation herangezogen. Die Synchronisationseinheit ist dazu geeignet und ausgebildet, den zeitlichen Verlauf des Beatmungssignals und den zeitlichen Verlauf des Diagnosesignals wenigstens teilweise auch unter Berücksichtigung aller untersuchten Signaländerungen in eine zeitliche Übereinstimmung zu bringen. Dadurch kann die Zuverlässigkeit der Synchronisierung überprüft und auch erheblich verbessert werden.

Es ist möglich, dass eine Synchronisierung verworfen wird, wenn eine bestimmte Anzahl der untersuchten Signaländerungen dabei nicht in zeitliche Übereinstimmung gebracht werden kann. Insbesondere wird eine Synchronisierung bestätigt bzw. behalten, wenn damit alle Signaländerungen oder zumindest ein überwiegender Teil der untersuchten Signaländerungen in eine plausible zeitliche Übereinstimmung gebracht werden können. Möglich ist auch, dass die Signaländerungen, welche bei einer erfolgten Synchronisierung nicht in zeitliche Übereinstimmung gebracht werden konnten, verworfen und für die weitere Analyse nicht herangezogen werden. Dann werden insbesondere nur solche zeitlichen Abschnitte im zeitlichen Verlauf synchronisiert und insbesondere auch weiter analysiert, welche im Bereich von Signaländerungen liegen, die sich in eine Synchronisierung einfügen.

Insbesondere ist die Synchronisationseinheit dazu geeignet und ausgebildet, eine Evaluierung der ermittelten Synchronität vorzunehmen und dazu zu untersuchen, wie viele der aufgefundenen Signaländerungen sich in eine vorgenommene Synchronisierung einreihen oder aus dieser herausfallen. Insbesondere kann bei Bedarf eine erneute Suche nach Signaländerungen und/oder eine erneute Synchronisation erfolgen. Möglich ist auch, dass ein Warnhinweis ausgegeben wird, dass die Zuverlässigkeit der Synchronisation unterhalb eines Schwellenwertes liegt.

Vorzugsweise wird in definierten Zeitbereichen nach paarweisen (auf demselben Ereignis beruhenden) Signaländerungen gesucht. Insbesondere sind die einzelnen Zeitbereiche voneinander beabstandet. Beispielsweise sind die Zeitbereiche 10 Minuten oder 30 Minuten oder eine oder mehrere Stunden beabstandet. So müssen nicht immer gesamte Verläufe miteinander verglichen werden. Falls in den vorgesehenen Zeitbereichen keine geeigneten Signaländerungen gefunden werden, können diese Zeitbereiche übersprungen werden oder die Abstände zwischen den Zeitbereichen werden verringert. Möglich ist auch, dass die Signalverläufe durchgehend nach paarweisen Signaländerungen durchsucht werden.

Insbesondere umfassen die Zeitbereiche einen Therapiebeginn und/oder ein Therapieende und/oder wenigstens einen zeitlich dazwischenliegenden liegenden Therapiebereich. So kann mit wenig Untersuchungsaufwand auch ein längerer Therapieverlauf synchronisiert werden. Therapiebeginn und/oder Therapieende umfassen z. B. die erste bzw. letzte Stunde. Möglich ist auch, dass zwischen Therapiebeginn und Therapieende eine Vielzahl von Zeitbereichen vorgesehen sind, in denen nach auf demselben Ereignis basierenden Signaländerungen gesucht wird.

Vorzugsweise umfassen die Zeitbereiche wenigstens 1 Minute und maximal 60 Minuten und vorzugsweise 15 Minuten +/- 5 Minuten. Beispielsweise sind die Zeitbereiche wenigstens 15 Minuten +/-5 Minuten oder auch länger voneinander beabstandet. Solche Zeitbereiche ermöglichen eine möglichst weitläufige Synchronisierung des Therapieverlaufs und können zugleich mit überschaubarem Analyseaufwand bzw. Rechenaufwand umgesetzt werden.

Die Synchronisationseinheit ist vorzugsweise dazu geeignet und ausgebildet, wenigstens eine Signaländerung zu erkennen, welche auf einem Ereignis beruht, welches aus einer Gruppe von Ereignistypen entnommen ist, umfassend: (akute) Atemstörung (Atemaussetzer, Husten), Bewegung des Patienten, verrutschte Atemschnittstelle, Leckage. Solche Ereignisse führen in der Regel zu besonders charakteristischen und gut identifizierbaren Signaländerungen in beiden Signalverläufen. Möglich sind auch andere physiologische bzw. durch den Patienten verursachte Ereignistypen.

Erfindungsgemäß ist die Synchronisationseinheit dazu geeignet und ausgebildet, Signaländerungen zu erkennen, welche durch wenigstens zwei unterschiedliche Ereignistypen bedingt sind. Dadurch kann die Reproduzierbarkeit der Synchronisation weiter verbessert werden. Erfindungsgemäß wird eine Plausibilitätskontrolle durchgeführt, ob die Signaländerungen der einzelnen Ereignistypen zu einem übereinstimmenden Ergebnis der Synchronisation führen oder nicht.

Das Beatmungssignal umfasst ein Maß für einen Fluss des Atemgases und/oder ein Maß für einen Druck des Atemgases oder ist ein solches. Das Beatmungssignal kann auch ein Maß für eine Leckagerate umfassen oder als ein solches ausgebildet sein. Insbesondere ist das Beatmungssignal ein Therapiedruck und/oder ein Atemfluss. Möglich ist auch, dass das Beatmungssignal Ableitungen oder andere mathematische Aufbereitungen eines solchen Maßes umfasst. Insbesondere ist die Signaländerung im zeitlichen Verlauf des Beatmungssignals eine Druckänderung und/oder eine Flussänderung und/oder eine Änderung der Leckagerate.

Das Diagnosesignal ist aus einer Gruppe von Diagnosesignaltypen entnommen, umfassend:
Blutgassensorsignale, EKG-Signale, EMG-Signale (z. B. Zwerchfell-EMG-Signale), Induktions-Plethysmografie-Signale, Blutdrucksensorsignale, (Körperschall-) Mikrofonsignale, Körperlagesensorsignale, Beschleunigungssensorsignale, Temperatursensorsignale, Druck- und/oder Flusssensorsignale, Videosignale, Wärmebildsignale, Radarsignale, Ultraschallsignale. Solche Diagnosesignale ermöglichen eine aussagekräftige Therapiekontrolle und eignen sich besonders vorteilhaft zur Analyse von Signaländerungen im Rahmen der Synchronisation. Das Diagnosesignal kann auch dem gleichen Typ wie das Beatmungssignal entsprechen (z. B. einem Therapiedruck oder dem Therapiefluss). Das Diagnosegerät ist insbesondere dazu geeignet und ausgebildet, solche Diagnosesignale zu erfassen.Es ist vorteilhaft und bevorzugt, dass mit der Erfassungseinrichtung wenigstens zwei für die Atemgasströmung charakteristische Beatmungssignaltypen erfassbar sind. Bevorzugt und vorteilhaft ist auch, dass mit der Sensoreinrichtung wenigstens zwei Diagnosesignaltypen erfassbar sind. Vorzugsweise ist in der Synchronisationseinheit wenigstens eine Zuordnung von wenigstens einem Beatmungssignaltyp zu wenigstens einem Diagnosesignaltyp hinterlegt. Die Synchronisationseinheit ist vorzugsweise dazu geeignet und ausgebildet, einen Signalverlauf eines Beatmungssignaltyps mit einem Signalverlauf eines gemäß der Zuordnung zugehörigen Diagnosesignaltyps zu synchronisieren. Zur Erfassung der Beatmungssignaltypen bzw. Diagnosesignaltypen werden insbesondere jeweils zugehörige Beatmungssignale bzw. Diagnosesignale erfasst. Insbesondere sind für die verschiedenen Typen jeweils bestimmte Sensormittel zur Erfassung bestimmter Messgrößen vorgesehen.

In einer besonders vorteilhaften Weiterbildung ist vorgesehen, dass das Ereignis wenigstens eine an dem Beatmungsgerät und/oder an dem Diagnosegerät ausgeführte Benutzereingabe ist. Vorzugsweise ist das Ereignis wenigstens eine zeitgleich am Beatmungsgerät und Diagnosegerät ausgeführte Benutzereingabe. Insbesondere wird durch die Benutzereingabe wenigstens ein Synchronisationssignal erzeugt. Insbesondere wird das Synchronisationssignal dem Beatmungssignal und/oder dem Diagnosesignal hinzugefügt. Insbesondere ist die Synchronisationseinheit dazu geeignet und ausgebildet, das Synchronisationssignal als eine Signaländerung im zeitlichen Verlauf des Beatmungssignals und/oder im zeitlichen Verlauf des Diagnosesignals zu erkennen und diese wenigstens eine Signaländerung für die Synchronisation heranzuziehen. Vorzugsweise wird der zeitliche Verlauf der Messgrößen nicht durch das Synchronisationssignal beeinflusst, um das Messergebnis bzw. die Therapiekontrolle nicht zu beeinflussen.

Zum Beispiel betätigt der Patient oder ein Betreuer am Beatmungsgerät und am Diagnosegerät zeitgleich eine Benutzerschnittstelle (z. B. Knopf oder Touchscreen). Durch ein solches Ereignis wird dann das Synchronisationssignal erzeugt und in beide Signalverläufe eingekoppelt. Die Synchronisationseinheit erkennt das Synchronisationssignal dann als Signaländerung und nutzt es bei der Synchronisation als zeitlichen Bezugspunkt in den Signalverläufen.

Im Rahmen der vorliegenden Erfindung wird unter einem Signalverlauf also nicht nur ein zeitlicher Verlauf der mittels der Sensoreinrichtung bzw. Erfassungseinrichtung gemessenen Größe verstanden, sondern insbesondere der über die Zeit registrierte Informationsgehalt. Beispielsweise kann das Synchronisationssignal als eine Information in einer Datei abgespeichert werden, in welcher auch der Verlauf der Messgrößen gespeichert wird. Im Rahmen der vorliegenden Erfindung wird unter einer Signaländerung vorzugsweise eine identifizierbare Markierung in den Signalverläufen verstanden, welche einen eindeutigen Zeitpunkt in den Signalverläufen markiert. Unter einer Signaländerung wird beispielsweise auch eine gezielt in einer Datei ergänzte Information verstanden, in welcher der Signalverlauf gespeichert ist. Das Beatmungssignal und/oder das Diagnosesignal können aber auch jeweils so ausgebildet sein, dass es dem von der Erfassungseinrichtung bzw. Sensoreinrichtung erfassten Messsignal direkt entspricht oder von diesem abgeleitet ist.

Als Reaktion auf ein Ereignis, beispielsweise eine Benutzereingabe, kann ein elektronisches und beispielsweise digitales Signal generiert werden. Ein solches Signal wird dann beispielsweise in die Signalverläufe eingebettet und/oder an die Signalverläufe vom Beatmungsgerät bzw. Diagnosegerät angebunden. Beispielsweise löst die Benutzereingabe einen (digitalen) Zeitstempel aus, welcher in den jeweiligen Signalverläufen identifizierbar ist und einen eindeutigen Zeitpunkt in den Signalverläufen markiert.

Zwischen dem Beatmungsgerät und dem Diagnosegerät sind vorzugsweise Daten übertragbar und besonders bevorzugt drahtlos übertragbar. Vorzugsweise ist das Ereignis ein Absenden und/oder ein Eintreffen eines zu übertragenden Datenpakets. Dabei ist die Synchronisationseinheit insbesondere dazu geeignet und ausgebildet, die Signalverläufe unter der Annahme zu synchronisieren, dass das Absenden und das Eintreffen des Datenpakets zeitgleich erfolgt oder unter einem definierten und bei der Synchronisation berücksichtigten Zeitversatz erfolgt ist. Insbesondere werden das Absenden und das Eintreffen des Datenpakets derart registriert, dass darüber ein eindeutiger Zeitpunkt definiert wird. Insbesondere wird der Zeitpunkt des Eintreffens des Datenpakets von dem Gerät registriert, welches das Datenpaket empfängt. Zugleich markiert dieses Gerät auch den Zeitpunkt in seinem eigenen Signalverlauf. Die Synchronisationseinheit kann dann die Signalverläufe über diesen eindeutigen Zeitpunkt synchronisieren. Bei einer ausreichend stabilen bzw. zügigen Datenübertragung wird so eine verlässliche und unaufwendige Synchronisation ermöglicht.

Beispielsweise sendet das Diagnosegerät seine Signalverläufe an das Beatmungsgerät. Das Beatmungsgerät registriert den Empfangszeitpunkt im vom Diagnosegerät gesendeten Signalverlauf und im eigenen Signalverlauf. Dadurch erhalten beide Signalverläufe einen Zeitstempel von derselben Uhr, im Beispiel also der Uhr des Beatmungsgerätes. Über einen solchen Zeitstempel kann die Synchronisationseinheit anschließend beide Signalverläufe gezielt und zuverlässig miteinander synchronisieren. Die drahtlose Übertragung erfolgt beispielsweise mittels Bluetooth, WLAN, NFC und/oder einer anderen geeigneten Übertragungstechnik.

Die Synchronisationseinheit ist insbesondere dazu geeignet und ausgebildet, für die Synchronisation eine Übertragungsqualität des Datenpakets zu berücksichtigen. Beispielsweise wird dazu eine Übertragungsrate und/oder eine Signalqualität oder dergleichen herangezogen. Die Synchronisationseinheit ist insbesondere dazu geeignet und ausgebildet, die Synchronisation zu verwerfen, falls die Übertragungsqualität unterhalb eines Schwellenwertes liegt. Möglich ist auch, dass in Abhängigkeit der Übertragungsqualität ein Zeitversatz zwischen Absenden und Eintreffen des Datenpakets einberechnet wird.

Es ist möglich, dass die Synchronisationseinheit dazu geeignet und ausgebildet ist, mittels der Beatmungseinrichtung die Atemgasströmung gezielt zu manipulieren, sodass im zeitlichen Verlauf des Beatmungssignals und im zeitlichen Verlauf des Diagnosesignals jeweils wenigstens eine wiedererkennbare Signaländerung auftritt, welche auf einem durch die Manipulation der Atemgasströmung verursachten zeitgleichen Ereignis beruhen. Die Manipulation umfasst insbesondere wenigstens eine gezielte Druckänderung und/oder Flussänderung der Atemgasströmung. Insbesondere ist die Manipulation eine für den Patienten sichere und unproblematische Beeinflussung der Atemgasströmung. Insbesondere ist die Manipulation im Rahmen der Therapie für den Patienten nicht nachteilig und insbesondere wahrnehmbar. Das ermöglicht eine konstruktiv unaufwendige und zugleich besonders zuverlässig identifizierbare Signaländerung und somit auch eine besonders genaue Synchronisation.

Bevorzugt umfasst das Beatmungssystem eine Synchronisationseinheit und ist dazu geeignet und ausgebildet, wenigstens eine Benutzereingabe und/oder wenigstens ein Absenden und Eintreffen eines übertragenden Datenpakets und/oder eine gezielte Manipulation der Atemgasströmung für die Synchronisation heranzuziehen. Insbesondere können alle hier beschriebenen Umsetzungen der Synchronisation jeweils alternativ oder in Kombination miteinander verwirklicht werden.

Die Synchronisationseinheit ist insbesondere dazu geeignet und ausgebildet, die synchronisierten Verläufe des Beatmungssignals und des Diagnosesignals auf einer gemeinsamen Zeitachse aufzutragen und insbesondere wenigstens einer Anzeigeeinrichtung und/oder Kommunikationseinheit bereitzustellen. Die Synchronisationseinheit kann dazu geeignet und ausgebildet sein, aus den synchronisierten zeitlichen Verläufen wenigstens eine Kennzahl zur Bewertung der Qualität der Beatmung zu ermitteln. Möglich ist auch, dass die Synchronisationseinheit in Abhängigkeit der Kennzahl Vorschläge für Änderungen ausgibt und/oder Änderungen an den Einstellungen der Beatmungseinrichtung vornimmt.

In allen Ausgestaltungen ist es bevorzugt, dass die Synchronisationseinheit in das Beatmungsgerät integriert ist. Dadurch muss im Rahmen einer (häuslichen) Therapiekontrolle lediglich ein koppelfähiges Diagnosegerät bereitgestellt werden, welches Selbst keine Synchronisationseinheit benötigt. Durch ein Einschalten von Beatmungsgerät und Diagnosegerät erfolgt dann insbesondere eine automatische Kopplung. Nach einem Start der Atemtherapie werden dann die entsprechenden Signalverläufe aufgezeichnet und später oder auch gleichzeitig von der Synchronisationseinheit synchronisiert und insbesondere weiter ausgewertet. Möglich und vorteilhaft ist auch, dass die Synchronisationseinheit in das Diagnosegerät integriert ist. In einer ebenfalls vorteilhaften Ausgestaltung kann vorgesehen sein, dass die Synchronisationseinheit wenigstens teilweise separat zum Beatmungsgerät und zum Diagnosegerät ausgebildet ist.

Die Sensoreinrichtung des Diagnosegeräts ist insbesondere wenigstens teilweise am Körper des Patienten anordenbar. Insbesondere umfasst die Sensoreinrichtung wenigstens eine Elektrode und wenigstens einen Elektrodenhalter. Das Diagnosegerät kann auch wenigstens eine Benutzerschnittstelle (Interface) zur Eingabe von subjektiven und/oder objektiven Patientendaten umfassen. Insbesondere umfasst die Sensoreinrichtung auch andere Arten von Sensormitteln.

Das Diagnosegerät kann wenigstens einen Effort-Gurt und/oder EIT-Gurt und/oder dergleichen umfassen. Zusätzlich oder alternativ kann ein für eine Induktions-Plethysmografie ausgebildeter Gurt vorgesehen sein. Das Diagnosegerät kann ein Zwerchfell-EMG-Gerät umfassen oder als ein solches ausgebildet sein. Die Sensoreinrichtung des Diagnosegeräts kann zur berührungslosen Erfassung ausgebildet sein, z. B. über Wärmebild, Radar und/oder Ultraschall. Insbesondere ist mit der Sensoreinrichtung des Diagnosegeräts eine für die Atemtätigkeit des Patienten charakteristische Größe messbar.

Das Verfahren (das nicht Teil der vorliegenden Erfindung ist) dient zum Betreiben eines Beatmungssystems, wie es zuvor beschrieben wurde. Auch ein solches Verfahren löst die zuvor gestellte Aufgabe besonders vorteilhaft. Insbesondere ist das Verfahren so ausgebildet, dass das Beatmungssystem auch in seinen Ausgestaltungen danach betrieben werden kann.

Das Beatmungsgerät ist insbesondere unabhängig vom Diagnosegerät betreibbar. Insbesondere ist das Beatmungsgerät auch ohne das Diagnosegerät bestimmungsgemäß zur Beatmung einsetzbar. Das Beatmungsgerät ist insbesondere ein Heimbeatmungsgerät. Es kann aber auch als ein klinisches Beatmungsgerät ausgebildet sein. Das Diagnosegerät und das Beatmungsgerät können direkt und/oder indirekt (z. B. über die Synchronisationseinheit oder eine externe Vorrichtung) miteinander koppelbar sein. Das Beatmungsgerät kann als ein Schlaftherapiegerät und insbesondere als ein CPAP-Gerät oder autoCPAP-Gerät oder auch als ein Highflow-Therapiegerät ausgebildet sein. Das Beatmungsgerät kann auch als eine andere Beatmungsgerätbauart ausgebildet sein.

Das über die Zeit erfasste Diagnosesignal wird insbesondere in einer Speichereinheit hinterlegt. Das über die Zeit erfasste Beatmungssignal wird insbesondere in einer Speichereinheit hinterlegt. Dazu können separate Speichereinheiten oder auch eine wenigstens gemeinsame Speichereinheit vorgesehen sein. Die gemeinsame Speichereinheit ist dann beispielsweise der Synchronisationseinheit oder dem Beatmungsgerät oder einer externen Vorrichtung zugeordnet.

Die Signalverläufe können eine sensorisch erfasste Größe direkt (Messgröße) oder eine von der erfassten Größe abgeleitete Größe betreffen. Für die Analyse zum Auffinden der Signaländerung können die Signalverläufe direkt und/oder deren Ableitung und/oder andere mathematische Aufbereitungen der Signalverläufe herangezogen werden.

In allen Ausgestaltungen ist es bevorzugt und vorteilhaft, dass die Beatmungseinrichtung unter Berücksichtigung des Beatmungssignals und/oder des Diagnosesignals ansteuerbar ist. Insbesondere ist in Abhängigkeit des Beatmungssignals und/oder des Diagnosesignals wenigstens eine Steuergröße für die Beatmung und/oder wenigstens eine Gerätefunktion einstellbar. In allen Ausgestaltungen ist es ebenfalls bevorzugt und vorteilhaft, dass wenigstens eine Steuergröße und/oder wenigstens eine Gerätefunktion des Diagnosegeräts unter Berücksichtigung des Beatmungssignals und/oder eines anderen Steuersignals des Beatmungsgeräts und/oder unter Berücksichtigung des Diagnosesignals einstellbar ist. Insbesondere werden die Signale des jeweils anderen Gerätes empfangen, um mindestens eine Funktion des empfangenden Gerätes zu steuern.

Beispielsweise empfängt das Beatmungsgerät wenigstens ein Signal vom Diagnosegerät. Möglich sind
- Z. B. Signale über die Atemtätigkeit zur Umschaltung von Exspirationsdruck und Inspirationsdruck oder kontinuierlichen Druckprofilen;
- Z. B. Signale aus einem EKG oder Defibrillator oder Beschleunigungssensor, um die Beatmung kurzzeitig zu unterbrechen, z. B. während Defibrillation oder Reanimation;
- Z. B. Signale über die Atemtätigkeit, um eine Zusatzfunktion synchron zur Atemphase zu steuern, bspw. Sauerstoff-Beimischung, Luftbefeuchtung, Verneblung, Aerosol-Abgabe;
- Z. B. Vitalsignale, um das Alarmsystem des Beatmungsgerätes für physiologische Alarme in Bezug auf die Signale des Diagnosegerätes zu nutzen, welches kein eigenes Alarmsystem besitzt;
- Z. B. eine Vielzahl von Signalen des Diagnosegerätes, um die telemedizinische Datenschnittstelle des Beatmungsgerätes zu nutzen, um Diagnosedaten zu übertragen;
- Z. B. eine Vielzahl von Signalen des Diagnosegerätes, um die Anzeigevorrichtung / Display des Beatmungsgerätes zur Anzeige der Diagnosesignale zu nutzen;
- Z. B. Signale über die Effektivität der Beatmung, z. B. Synchronität oder Blutgase, um Beatmungsparameter zu steuern, z. B. die Höhe der Druckniveaus, die Inspirationszeit, die Exspirationszeit oder die Empfindlichkeit des Triggers.

Beispielsweise empfängt das Diagnosegerät Signale vom Beatmungsgerät. Möglich sind
- Z. B. Signale über die Atemtätigkeit zur Atemphase zum Auslösen einer Elektrostimulation, z. B. von Atemmuskeln, dem Herz oder Muskeln zur Öffnung der Atemwege;
- Z. B. eine Vielzahl von Signalen des Beatmungsgerätes, um die telemedizinische Datenschnittstelle des Diagnosegerätes zu nutzen, um Beatmungsdaten zu übertragen;
- Z. B. eine Vielzahl von Signalen des Beatmungsgerätes, um die Anzeigevorrichtung / Display des Diagnosegerätes zur Anzeige der Beatmungssignale zu nutzen.

Insbesondere stellen die zuvor beispielhaft genannten Signale Beatmungssignale bzw. Diagnosesignale dar.

Das Diagnosegerät kann auch ein Modul mit einem oder mehreren Sensoren im Atemgasstrom sein. Die Sensoren im Atemgasstrom können beispielsweise zur Messung der O2-Konzentration und/oder der CO2-Konzentration und/oder der Temperatur und/oder der absoluten Feuchte und/oder der relativen Feuchte des Atemgasstroms eingerichtet sein. Die Sensoren im Atemgasstrom können beispielsweise auch zur Messung von volatilen organischen Verbindungen, kurz VOCs, im Atemgasstrom eingerichtet sein.

Durch die Synchronisierung kann beispielsweise eine Auswertung nach Atemphasen ermöglicht werden, also nach Inspiration und Exspiration. Durch die Synchronisierung kann auch eine Auswertung nach Teilen der Atemphasen ermöglicht werden, wie beispielsweise dem Ende der Exspiration.

Insbesondere ist die Synchronisationseinheit dazu geeignet und ausgebildet, die im Rahmen der vorliegenden beschreibungsverfahrensartig formulierten Merkmale auszuführen. In der Synchronisationseinheit ist dazu insbesondere wenigstens ein Algorithmus hinterlegt. Insbesondere umfasst die Synchronisationseinheit wenigstens eine elektronische Rechnereinheit.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Beatmungssystems;
- Fig. 2: ein stark schematisiertes Schaubild mit Therapiephasen und Diagnosephasen;
- Fig. 3: eine rein schematische Auftragung von Beatmungssignalen und Therapiesignalen über die Zeit;
- Fig. 4: eine rein schematische Auftragung der Beatmungssignale und Therapiesignale aus der Fig. 3 nach einer von dem Beatmungssystem durchgeführten Synchronisation; und
- Fig. 5: ein vergrößerter Ausschnitt der Auftragung aus der Fig. 4.

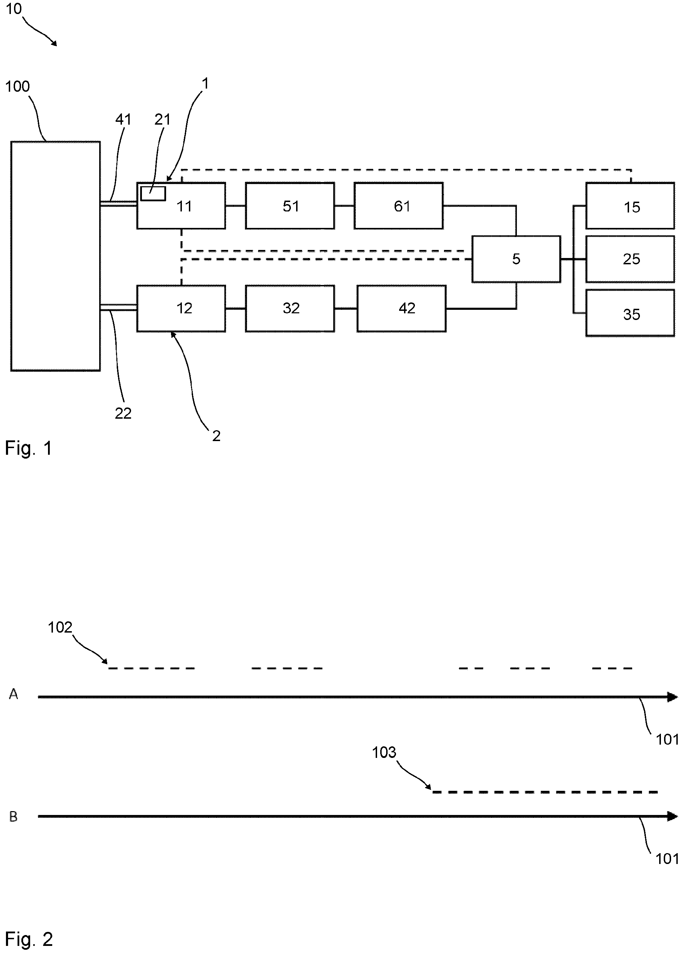

Die Figur 1 zeigt ein erfindungsgemäßes Beatmungssystem 10 mit einem Beatmungsgerät 1 und einem mit dem Beatmungsgerät 1 gekoppelten Diagnosegerät 2. Des Beatmungsgerät 1 weist eine Beatmungseinrichtung 11 auf, welche mittels einer Lüftereinrichtung oder einer Druckgasquelle eine Atemgasströmung erzeugt und dem Patienten 100 zur Beatmung über eine Atemschnittstelle 41 zuführt. Die Atemschnittstelle 41 ist z. B. eine Atemmaske, welche über eine Schlaucheinrichtung mit der Beatmungseinrichtung 11 verbunden ist.

Alternativ zur Atemmaske können auch andere Patientenschnittstellen verwendet werden, z. b. ein Traceostoma (bzw. anderes invasives Interface) oder Highflow-Interface. Die Beatmungseinrichtung 11 kann auch in der Maske integriert sein. Optional können ein Befeuchter zwischen Beatmungsgerät 1 und Patient 100 und/oder eine Einrichtung zur Einleitung von Sauerstoff bzw. Aerosolen und Medikamenten vorgesehen sein.

Das Beatmungsgerät 1 kann nicht-invasiv oder invasiv ausgebildet sein. Darunter werden z. B. auch Geräte 1 zur Schlaftherapie (CPAP, autoCPAP, Bilevel, ASV-Therapie) oder nasalen Highflow-Therapie verstanden. Das Gerät 1 enthält hier auch ein User-Interface und Anschlüsse für Strom und Verbindung zum Patienten 100.

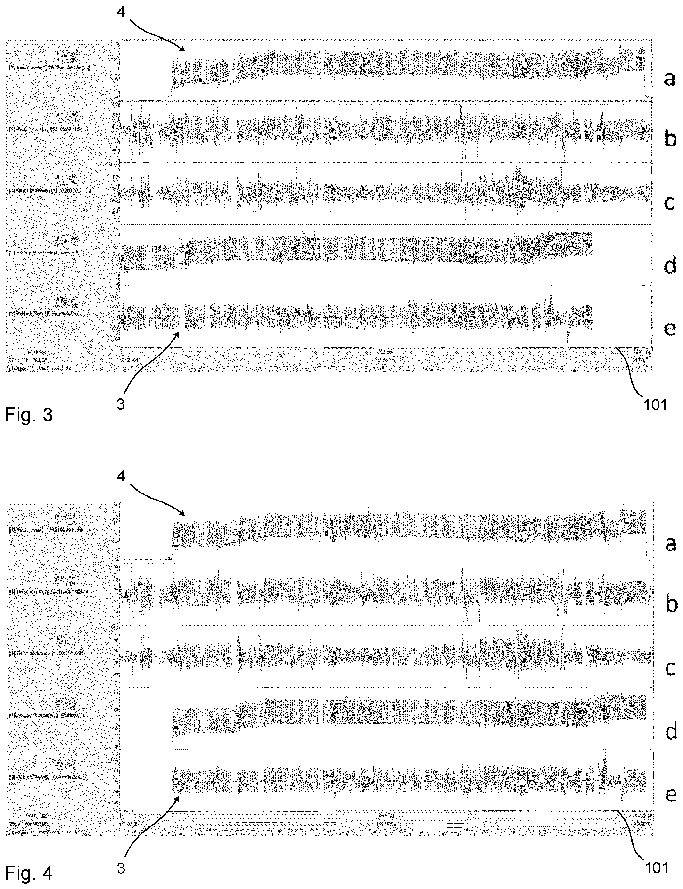

Das Beatmungsgerät 1 umfasst eine Erfassungseinrichtung 21 mit Sensormitteln zur Erfassung wenigstens eines mit Bezug zu der Fig. 3 vorgestellten Beatmungssignals 3 über die Zeit. Vorzugsweise werden Beatmungssignale 3 von zwei oder mehr Beatmungssignaltypen 3 erfasst. Die Beatmungssignale 3 sind charakteristisch für die Atemgasströmung und betreffen z. B. wenigstens ein Drucksignal und ein Flusssignal. Beispielsweise werden dadurch der Therapiedruck und der Atemfluss sowie eine Leckagerate fortlaufend überwacht. Zudem weist das Beatmungsgerät 1 hier einen Controller bzw. eine Steuereinrichtung zur Verarbeitung der Signale und zur Steuerung der Beatmungseinrichtung 11 auf.

Die Beatmungssignalverläufe werden hier in einer innerhalb des Beatmungsgerätes 1 angeordneten Speichereinheit 51 registriert. Beispielsweise sind ein Arbeitsspeicher, permanenter Speicher und/oder ein tragbares Speichermedium vorgesehen. Die Beatmungssignale 3 werden zwischengespeichert und mit einem Zeitstempel gemäß einer internen Uhr versehen.

Dazu ist mindestens eine interne Uhr (RTC) vorgesehen. Bevorzugt kann diese interne Uhr unabhängig oder teilweise unabhängig von der am Userinterface angezeigten Zeit gestellt werden - sodass der Anwender bzw. Patient die Uhrzeit so stellen kann, wie er möchte, und das interne Messverhalten des Beatmungsgerätes 1 und des gesamten Systems 10 nicht davon abhängig ist.

Das Beatmungsgerät 1 ist hier mit einer Kommunikationseinheit 61 zur Übermittlung der aufgezeichneten Signale 3 ausgestattet. Beispielsweise umfasst die Kommunikationseinheit 61 Kabelverbindungen (seriell, Netzwerk, HL7, PDMS, I2C, USB, Firewire, etc.), mobile Speichermedien (Speicherkarte, USB-Stick, etc.), drahtlose Kurzstreckenkommunikation (Bluetooth, Infrarot), drahtlose Langstreckenkommunikation (GSM, LPWA, 3G, 4G, 5G, Richtfunk, Sigfox, Lora). Zusätzlich oder alternativ kann eine wenigstens teilweise weitere Verarbeitung der Daten im Beatmungsgerät 1 selbst stattfinden.

Das Diagnosegerät 2 dient hier zur Kontrolle der Atemtherapie. Dazu wird das Diagnosegerät 2 bedarfsweise mit dem Beatmungsgerät 1 wirkverbunden, sodass während der Therapie Parameter erfasst werden können, welche dann eine Bewertung der Therapiequalität erlauben. Dazu umfasst das Diagnosegerät 2 hier eine Sensoreinrichtung 12 zur Erfassung von Diagnosesignalen 4 verschiedener Diagnosesignaltypen, welche in einer Speichereinheit 32 des Diagnosegeräts 2 über die Zeit registriert werden. Die Diagnosesignale 4 werden mit Bezug zu der Fig. 3 näher vorgestellt. Die Diagnosesignale 4 werden zwischengespeichert und können mit einem Zeitstempel gemäß einer internen Uhr versehen werden.

Zur Verbindung mit dem Patienten 100 umfasst das Diagnosegerät 2 mehrere Diagnoseschnittstellen 22, von denen hier nur eine exemplarisch dargestellt ist. Die Diagnoseschnittstellen 22 werden durch Sensoren der Sensoreinrichtung 12 bereitgestellt. Beispielsweise umfasst die Sensoreinrichtung 12 Elektroden, Effort-Gurte, EIT-Gurte, Sensoren zur Messung von Blutgasen, Körperschallmikrofone, Lagesensoren, Beschleunigungssensoren, Druck- oder Flusssensoren, Temperatursensoren, Blutdruck-Sensoren, EKG-Sensoren, EMG-Sensoren, optische Sensoren, elektrische Sensoren, chemische Sensoren. Zusätzlich oder alternativ zu einem Effort-Gurt kann ein für eine Induktions-Plethysmografie ausgebildeter Gurt vorgesehen sein. Zudem kann ein Empfang von Bildern (Videokamera) oder Sprache (Mikrofon) vorgesehen sein. Es kann auch ein User-Interface zur Eingabe von Werten, z. B. Ausfüllen von Fragebögen zu Symptomen, Lebensqualität, Nebenwirkungen und Problemen vorhanden sein.

Das Diagnosegerät 2 kann ein User-Interface für weitere Eingaben/Ausgaben, Anschlüsse für Strom und Verbindung zum Patienten 100, eine Batterie, einen Akku, einen Controller bzw. eine Steuereinrichtung zur Verarbeitung der Signale 4 und/oder Steuerung der Komponenten des Diagnosegerätes 2 und/oder wenigstens eine interne Uhr (RTC) aufweisen.

Das Diagnosegerät 2 ist hier mit einer Kommunikationseinheit 42 zur Übermittlung der aufgezeichneten Signale 4 ausgestattet. Die Kommunikationseinheit 42 ist beispielsweise wie die Kommunikationseinheit 61 des Beatmungsgeräts 1 ausgebildet. Zusätzlich oder alternativ kann eine wenigstens teilweise weitere Verarbeitung der Daten im Diagnosegerät 2 selbst stattfinden. Auch die Speichereinheit 32 des Diagnosegeräts 2 kann wie die Speichereinheit 51 des Beatmungsgeräts 1 ausgebildet sein.

Bei Bedarf können auch zwei oder mehr Diagnosegeräte 2 am Patienten 100 angebunden und mit dem Beatmungsgerät 1 gekoppelt sein.

Um die Beatmungssignale 3 und die Diagnosesignale 4 für die Therapiekontrolle auswerten zu können, müssen diese in der Regel miteinander verglichen werden. Dazu ist es hilfreich und oft auch zwingend notwendig, die Signale 3, 4 in eine zeitliche Übereinstimmung (Synchronität) zu bringen bzw. zu synchronisieren. Nach einer erfolgten Synchronisation kann dann beispielsweise ein Arzt und/oder auch ein Auswertealgorithmus des Beatmungssystems 10 in den Signalverläufen erkennen, welche besonderen Vorkommnisse in der Atemtätigkeit des Patienten zeitgleich mit anderen Geschehnissen aufgetreten sind, beispielsweise mit Veränderungen des Blutdrucks oder der Herzfrequenz oder dergleichen. Darüber können besonders verlässlich eine Diagnose getroffen und die Beatmungseinstellungen besser an die Bedürfnisse angepasst werden.

Um die Synchronisation automatisiert auszuführen, umfasst das Beatmungssystem 10 eine Synchronisationseinheit 5, welche mit dem Beatmungsgerät 1 und dem Diagnosegerät wirkverbunden ist. In dem hier gezeigten Beispiel kann die Synchronisationseinheit 5 dazu mit den Kommunikationseinheiten 42, 61 Daten austauschen.

Die Synchronisationseinheit 5 des Beatmungssystems 10 der Fig. 1 und ihre Funktionsweise werden nachfolgend mit Bezug zu den Figuren 2 bis 5 näher vorgestellt.

Die Synchronisationseinheit 5 ermittelt hier z. B. mindestens einmal, idealerweise wiederholt, den Zeitversatz der Signale 3, 4 und korrigiert diese, sodass diese anschließend zeitsynchron mit einem Versatz kleiner 1 s weiterverarbeitet bzw. ausgewertet werden können.

Die Synchronisationseinheit 5 erzeugt oder empfängt ein Referenz-Zeitsignal (kann auch als Datenpaket oder Zeitstempel bezeichnet werden), überträgt dies in Quasi-Echtzeit gemäß der gestrichelten Linien in Fig. 1 an 11 und 12. Die beiden Geräte 1, 2 verwenden dieses Referenzsignal zum Stellen ihrer internen Uhrzeit (RTC). Dies ist nur möglich, wenn eine permanente Verbindung zwischen der Synchronisationseinheit 5 und den Geräten 1, 2 in Quasi-Echtzeit besteht. Das Ereignis 6 ist in einem solchen Fall dann die Übertragung des Datenpakets und die damit verbundene Uhrzeit.

Die Synchronisationseinheit 5 wertet die Zeitstempel der einzelnen Geräte 1, 2 aus, ermittelt die Differenz aus beiden und korrigiert mindestens einen der Zeitstempel derart, dass für die weitere Verarbeitung der Signale 3, 4 ein Zeitversatz kleiner 1 s besteht. Hierfür ist mindestens einmalig eine Verbindung in Quasi-Echtzeit zwischen den Geräten 1, 2 und der Synchronisationseinheit 5 nötig.

Ein Signal 3, 4, z. B. der Therapiedruck, wird von beiden Geräten 1, 2 aufgezeichnet. Anhand einer Ähnlichkeitsanalyse des Signals 3, 4 erkennt die Synchronisationseinheit 5 den Zeitversatz und korrigiert die Zeitstempel mindestens eines der beiden Geräte 1, 2 derart, dass der Zeitversatz auf weniger als eine Sekunde reduziert wird. Die Ähnlichkeitsanalyse kann z. B. durch eine Korrelation, einen Matching Pursuit Vergleich, einen Abgleich der Ein- und Ausschaltzeiten oder eine Minimierung der Fehlersumme bzw. Summe der Fehlerquadrate erfolgen.

Eine Information, z. B. die Atmungstätigkeit, wird von unterschiedlichen in beiden Geräten 1, 2 aufgezeichnet, z. B. im Beatmungsgerät 1 durch einen Fluss-Sensor und im Diagnosegerät 2 durch einen Effort-Sensor. Anhand einer Ähnlichkeitsanalyse des Signals 3, 4 erkennt die Synchronisationseinheit 5 den Zeitversatz und korrigiert die Zeitstempel mindestens eines der beiden Geräte 1, 2 derart, dass der Zeitversatz auf weniger als eine Sekunde reduziert wird. Die Ähnlichkeitsanalyse kann z. B. durch eine Korrelation, einen Matching Pursuit Vergleich, einen Abgleich der Ein- und Ausschaltzeiten oder eine Minimierung der Fehlersumme bzw. Summe der Fehlerquadrate erfolgen. Dabei wird die Phasenlage der unterschiedlichen Atemsignale aufgrund der unterschiedlichen verwendeten Sensoren erkannt und korrigiert. Z. B. entspricht der Beginn der Einatmung einem lokalen Minimum im Effortsignal und einem Nulldurchgang im Atemfluss-Signal.

Beide Geräte 1, 2 erhalten mindestens einmalig, bevorzugt in regelmäßigen Abständen, ein Zeitsignal von einem Zeitgeber, z. B. per Funk, und richten ihre interne RTC Uhr danach aus.

Die Geräte 1, 2 sind hier derart miteinander verbunden, dass eines von beiden zu charakteristischen Zeitpunkten, z. B. bei Messbeginn, einen Zeit-Marker an das andere Gerät 1, 2 oder die Synchronisationseinheit 5 sendet, anhand dessen die ZeitStempel verglichen und korrigiert werden können.

Dabei verändert sich der Zeitversatz häufig über die Zeit aufgrund der unterschiedlichen Geschwindigkeiten der inneren Uhren (RTC) von Gerät 1 und 2. Dies wird idealerweise ebenfalls ausgeglichen. Dazu wird der Unterschied zwischen beiden Zeitstempeln nach einer der genannten Methoden wiederholt durchgeführt und die Gangunterschiede neu ermittelt, um sie im Anschluss auszugleichen. Dadurch entstehen unterschiedlich viele Abtastwerte der Signale 3, 4 der Geräte 1, 2. Dies wird z. B. durch Halten eines Abtastwertes bzw. Interpolation von Abtastwerten derart ausgeglichen, dass die Signale im Anschluss an die Synchronisationseinheit 5 zeitsynchron verlaufen und wieder gleich viele Abtastschritte aufweisen.

Die Synchronisationseinheit 5 ist hier mit einer Regeleinheit 15 ausgestattet. Diese ändert den Betriebszustand des Beatmungsgerätes 1 basierend auf der Summe der zeitsynchronen Signale beider Geräte 1, 2. Z. B. aufgrund manueller Steuerung oder durch eine automatische Steuerung, die z. B. Asynchronien auf Basis der Effortsignale von Gerät 2 und des Druck-Signals von Gerät 1 erkennt und basierend darauf die Einstellungen der Beatmungseinrichtung 11 ändert, z. B. die Trigger-Sensitivität oder die Cycling-Sensitivität oder die Inspirationszeit oder die Exspirationszeit mit dem Ziel, die Synchronität zwischen Beatmungsgerät 1 und Patient 100 zu verbessern. Alternativ können exspiratorische oder inspiratorische Flusslimitation aufgrund von Effortsignalen oder EMG-Signalen oder EIT-Signalen in Kombination mit dem Atemfluss-Signal des Beatmungsgerätes 1 erkannt und durch eine Änderung mindestens eines Druckniveaus oder mindestens einer Steigung des Überganges zwischen inspiratorischem und exspiratorischem Druck reduziert werden.

Eine Anzeigeeinheit 25 zeigt die zeitsynchronen Signale 3, 4 aus Beatmungsgerät 1 und Diagnosegerät 2 an.

Eine Speichereinheit 35 speichert die zeitsynchronen Signale 3, 4 aus Beatmungsgerät 1 und Diagnosegerät 2 ab. Optional versendet die Speichereinheit 35 die Signale 3, 4 an eine Gegenstelle, z. B. ein Server oder PC oder ein mobiles Endgerät.

Die Synchronisationseinheit 5 und insbesondere ihre Komponenten 15, 25, 35 können einzeln oder gemeinsam in einem der beiden Geräte 1 oder 2 bzw. in einem PC oder Server oder mobilen Endgerät oder in einer speziell zu diesem Zweck hergestellten Auswertungseinrichtung als Module integriert sein.

In der Figur 2 ist ein beispielhafter Therapieverlauf mit einer Therapiekontrolle gezeigt. Auf der Achse A sind Therapiephasen 102 (gestrichelte Linien) und Abschnitte ohne Therapie (keine gestrichelten Linien) über die Zeit 101 aufgetragen. Typischerweise verbleibt das Beatmungsgerät 1 über einen längeren Zeitraum, mindestens mehrere Tage, am Patienten 100 und es finden wiederholt Therapie-Sessions statt. Zusätzlich zu den Signaldaten werden auch Statistikdaten gespeichert, z. B. Nutzungsdauer pro Tag oder Mediane von Atemfrequenz, Leckage, Tidalvolumen, Minutenvolumen pro Tag oder die Anzahl von Ereignissen wie Apnoen oder Asynchronien pro Tag oder pro Stunde gespeichert.

Auf der Achse B sind mit dem Diagnosegerät 2 durchgeführte Diagnosephasen 103 (gestrichelte Linien) und Abschnitte ohne Diagnosegerät 2 (keine gestrichelten Linien) über die Zeit 101 aufgetragen. Das Diagnosegerät 2 verbleibt meist nur eine kürzere Zeit zur Therapiekontrolle beim Patienten 100. Häufig nur für einen Tag / eine Nacht oder einige wenige Tage und Nächte. Daher befinden sich im Speicher 32 oft Daten zu weniger Therapietagen als im Beatmungsgerät 1. Die Start- und Endzeitpunkte der Diagnosemessungen sind typischerweise nicht synchron mit den Start- und Endzeitpunkten der Therapiemessungen. Dies kann zusätzlich durch unterschiedlich gestellte interne Uhren (RTC) verstärkt werden. Daher sind hier auch zwei Zeitskalen gezeichnet. Typischerweise sind die Daten aus den Beatmungsgeräten 1 öfter unterbrochen, z. B. durch Toilettengänge, als die des Diagnosegerätes 2, sodass mehrere Messabschnitte (bzw. Signalverläufe 3) des einen Gerätes 1 mit einem Messabschnitt (bzw. Signalverlauf 4) des anderen Gerätes 2 verbunden werden müssen.

Hier ist die Synchronisationseinheit 5 in der Lage, die Gesamtheit der gespeicherten Information zu verarbeiten, speichern, darzustellen und als Basis für eine Optimierung der Beatmungseinstellungen zu nutzen. D. h., für weiter zurückliegende Tage liegen häufig nur Statistikdaten und manchmal auch Signaldaten aus dem Beatmungsgerät 1 vor, für die Tage der Therapiekontrolle zusätzlich noch Daten aus dem Diagnosegerät 2. Entsprechend werden für diese Tage zusätzlich Signale 3, 4 verarbeitet und dargestellt.

In der Figur 3 sind beispielhafte Beatmungssignale 3 und Diagnosesignale 4 über die Zeit 101 aufgetragen, welche während einer Therapiekontrolle erfasst wurden. Die Signale 3, 4 sind hier im Originalzustand, also nicht zeitsynchron. Die Diagnosesignale 4 umfassen hier drei Diagnosesignaltypen: Therapiedruck (a), Effortgurt Thorax (b), Effortgurt Abdomen (c). Die Beatmungssignale 3 umfassen hier zwei Beatmungssignaltypen: Therapiedruck (d), Atemfluss (e). Es ist an den beiden Therapiedruck-Signalen (a, d) gut zu erkennen, dass die Aufzeichnungen in Bezug auf die zeit 101 gegeneinander verschoben sind.

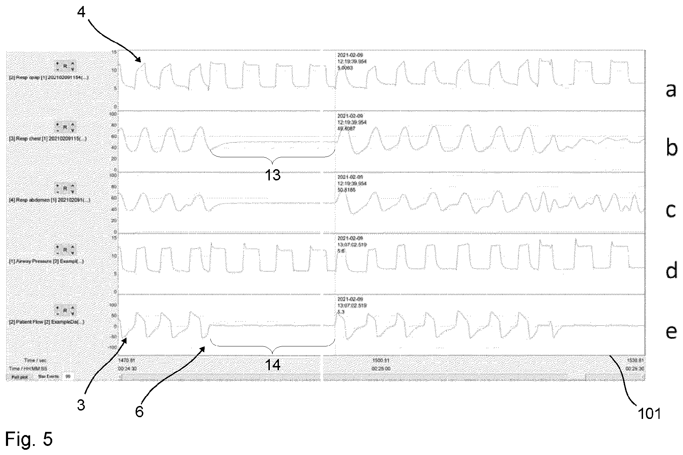

Die Figur 4 zeigt die Beatmungssignale 3 und Diagnosesignale 4 der Figur 3, nachdem sie durch die Synchronisationseinheit 5 synchronisiert wurden. Zur besseren Übersicht zeigt die Figur 5 eine ausschnittsweise Vergrößerung der Figur 4.

Hier wurden die Beatmungssignale 3 auf der Zeitachse verschoben, um mit den Diagnosesignalen 4 synchron zu laufen. Besonders an den Druckverläufen (a, d) ist die korrekte zeitliche Zuordnung besonders gut erkennen. Für die Synchronisation wurden hier jedoch das Signal des Thorax-Effortgurts (b) des Diagnosegerätes 2 und das Atemfluss-Signal (e) des Beatmungsgerätes 1 verwendet. In der Fig. 5 ist die hohe zeitliche Synchronität nach der Korrektur mit einem verbleibenden Versatz kleiner 1 s gut zu erkennen.

Für die hier gezeigte Synchronisation hat die Synchronisationseinheit 5 im zeitlichen Verlauf des Beatmungssignals 3 und im zeitlichen Verlauf des Diagnosesignals 4 Signaländerungen 13, 14 identifiziert, welche durch dasselbe Ereignis 6 verursacht wurden. Solche Signaländerungen 13, 14 sind in der Figur 5 beispielhaft für die Signaltypen b und e markiert. Durch dasselbe Ereignis 6 (z. B. ein Atemaussetzer oder eine Leckage aufgrund einer Maskenundichtigkeit) kam es in beiden Signalen zu einem signifikant linearen Verlauf mit einer Steigung von etwa null. Beginn und Ende des Ereignisses 6 sind durch die kurzfristigen Veränderungen der Steigung deutlich zu identifizieren.

Ausgehend von diesen Signaländerungen 13, 14 hat die Synchronisationseinheit 5 auch die übrigen Verläufe von Beatmungssignalen 3 und Diagnosesignalen 4 in eine zeitliche Übereinstimmung gebracht. Ein Vergleich von anderen Signaländerungen 13, 14 im Verlauf und z. B. der Maxima und Minima zeigt, dass auch diese in sehr genauer Überstimmung sind. Die Synchronisation ist über den gesamten Verlauf plausibel.

Für die Synchronisation kann die Synchronisationseinheit 5 auch gezielt Signaländerungen 13, 14 suchen, welche auf anderen als den zuvor beschriebenen Ereignissen 6 beruhen. Beispielsweise können den Beatmungssignalen 3 und/oder Diagnosesignalen 4 während ihrer Aufzeichnung Signaländerungen 13, 14 zugewiesen werden, welche durch eine Benutzereingabe an den Geräten 1, 2 ausgelöst werden. So kann durch die Benutzereingabe den Signalverläufen aus beiden Geräten 1, 2 jeweils ein Synchronisationssignal hinzugefügt werden, welches einen eindeutigen Zeitpunkt markiert. Beispielsweise können auch durch das Absenden und/oder Eintreffen der übertragenden Datenpakete mit den Signalen 3, 4 gezielt Signaländerungen (z. B. Zeitstempel) erzeugt werden, welche die Synchronisationseinheit 5 dann zur Synchronisation sucht und als Bezugspunkte nutzt. Solche Signaländerungen 13, 14 sind hier nicht dargestellt und können z. B. in eine Datei des Signalverlaufs eingebettet sein.

### Bezugszeichenliste:

- 1: Beatmungsgerät
- 2: Diagnosegerät
- 3: Beatmungssignal
- 4: Diagnosesignal
- 5: Synchronisationseinheit
- 6: Ereignis
- 10: Beatmungssystem
- 11: Beatmungseinrichtung
- 12: Sensoreinrichtung
- 13: Signaländerung
- 14: Signaländerung
- 15: Regeleinheit
- 21: Erfassungseinrichtung
- 22: Diagnoseschnittstelle
- 25: Anzeigeeinheit
- 32: Speichereinheit
- 35: Speichereinheit
- 41: Atemschnittstelle
- 42: Kommunikationseinheit
- 51: Speichereinheit
- 61: Kommunikationseinheit
- 100: Patient
- 101: Zeit
- 102: Therapiephase
- 103: Diagnosephase

## Patentansprüche

1. Beatmungssystem (10) mit wenigstens einem Beatmungsgerät (1) und mit wenigstens einem Diagnosegerät (2), wobei das Beatmungsgerät (1) wenigstens eine Beatmungseinrichtung (11) zur Erzeugung einer Atemgasströmung für eine Beatmung und wenigstens eine Erfassungseinrichtung (21) zur Erfassung wenigstens eines für die Atemgasströmung charakteristischen Beatmungssignals (3) über die Zeit umfasst und wobei das Diagnosegerät (2) wenigstens eine Sensoreinrichtung (12) zur Erfassung wenigstens eines Diagnosesignals (4) über die Zeit umfasst, wobei wenigstens eine Synchronisationseinheit (5) mit der Erfassungseinrichtung (21) und der Sensoreinrichtung (12) wirkverbunden ist und wobei die Synchronisationseinheit (5) dazu geeignet und ausgebildet ist, wenigstens einen zeitlichen Verlauf des Beatmungssignals (3) und wenigstens einen zeitlichen Verlauf des Diagnosesignals (4) jeweils auf wenigstens eine durch dasselbe Ereignis (6) verursachte Signaländerung (13, 14) zu untersuchen und den Verlauf des Beatmungssignals (3) und den Verlauf des Diagnosesignals (4) so in eine zeitliche Übereinstimmung zu bringen, dass das Ereignis (6) in beiden Signalverläufen zeitgleich auftritt, wobei das Beatmungssignal (3) ein Maß für einen Fluss des Atemgases und/oder ein Maß für einen Druck des Atemgases und/oder ein Maß für eine Leckagerate umfasst und das Diagnosesignal (4) aus einer Gruppe von Diagnosesignaltypen entnommen ist, umfassend:
Blutgassensorsignale, EKG-Signale, EMG-Signale, Induktions-Plethysmografie-Signale, (Körperschall-)Mikrofonsignale, Körperlagesensorsignale, Beschleunigungssensorsignale, Temperatursensorsignale, Videosignale, Wärmebildsignale, Radarsignale, Ultraschallsignale, wobei die Synchronisationseinheit (5) dazu geeignet und ausgebildet ist, den zeitlichen Verlauf des Beatmungssignals (3) und den zeitlichen Verlauf des Diagnosesignals (4) jeweils auf eine Mehrzahl von Signaländerungen (13, 14) zu untersuchen, welche jeweils paarweise auf demselben Ereignis (6) beruhen und den zeitlichen Verlauf des Beatmungssignals (3) und den zeitlichen Verlauf des Diagnosesignals (4) wenigstens teilweise auch unter Berücksichtigung der weiteren Signaländerungen (13, 14) in eine zeitliche Übereinstimmung zu bringen, **dadurch gekennzeichnet, dass** die Synchronisationseinheit (5) dazu geeignet und ausgebildet ist, Signaländerungen (13, 14) zu erkennen, welche durch wenigstens zwei unterschiedliche Ereignistypen bedingt sind und zudem eine Plausibilitätskontrolle durchzuführen, ob die Signaländerungen der einzelnen Ereignistypen zu einem übereinstimmenden Ergebnis der Synchronisation führen oder nicht.

2. Beatmungssystem (10) nach dem vorhergehenden Anspruch, wobei die Synchronisationseinheit (5) dazu geeignet und ausgebildet ist, durch die durch dasselbe Ereignis (6) verursachten Signaländerungen (13, 14) wenigstens einen eindeutigen Zeitpunkt im zeitlichen Verlauf des Beatmungssignals (3) und im zeitlichen Verlauf des Diagnosesignals (4) zu erkennen und die Signalverläufe auf diesen Zeitpunkt zu synchronisieren.

3. Beatmungssystem (10) nach einem der vorhergehenden Ansprüche, wobei die Synchronisationseinheit (5) dazu geeignet und ausgebildet ist, ein Maß für eine Ähnlichkeit der Signaländerungen (13, 14) im zeitlichen Verlauf des Beatmungssignals (3) und im zeitlichen Verlauf des Diagnosesignals (4) zu ermitteln und in Abhängigkeit der Ähnlichkeit zu bestimmen, ob die Signaländerungen (13, 14) auf demselben Ereignis (6) beruhen oder nicht.

4. Beatmungssystem (10) nach dem vorhergehenden Anspruch, wobei das Maß für die Ähnlichkeit eine Dauer und/oder eine Intensität und/oder eine Symmetrieeigenschaft ist.

5. Beatmungssystem (10) nach dem vorhergehenden Anspruch, wobei in definierten Zeitbereichen nach paarweisen Signaländerungen (13, 14) gesucht wird.

6. Beatmungssystem (10) nach dem vorhergehenden Anspruch, wobei die Zeitbereiche insbesondere einen Therapiebeginn und ein Therapieende und wenigstens einen zeitlichen dazwischen liegenden Therapiebereich umfassen.

7. Beatmungssystem (10) nach einem der beiden vorhergehenden Ansprüche, wobei die Zeitbereiche wenigstens 1 Minute und maximal 60 Minuten und vorzugsweise 15 Minuten +/- 5 Minuten umfassen.

8. Beatmungssystem (10) nach einem der vorhergehenden Ansprüche, wobei die Synchronisationseinheit (5) dazu geeignet und ausgebildet ist, wenigstens eine Signaländerung (13, 14) zu erkennen, welche auf einem Ereignis (6) beruht, welches aus einer Gruppe von Ereignistypen entnommen ist, umfassend: (akute oder chronische) Atemstörung (Atemaussetzer, Husten), Bewegung des Patienten, verrutschte Atemschnittstelle (41), Leckage.

9. Beatmungssystem (10) nach einem der vorhergehenden Ansprüche, wobei mit der Erfassungseinrichtung (21) wenigstens zwei für die Atemgasströmung charakteristische Beatmungssignaltypen erfassbar sind und/oder wobei mit der Sensoreinrichtung (12) wenigstens zwei Diagnosesignaltypen erfassbar sind und wobei in der Synchronisationseinheit (5) eine Zuordnung von wenigstens einem Beatmungssignaltyp zu wenigstens einem Diagnosesignaltyp hinterlegt ist und wobei die Synchronisationseinheit (5) dazu geeignet und ausgebildet ist, einen Signalverlauf (3) eines Beatmungssignaltyps mit dem Signalverlauf (4) eines gemäß der Zuordnung zugehörigen Diagnosesignaltyps zu synchronisieren.

10. Beatmungssystem (10) nach einem der vorhergehenden Ansprüche, wobei das Ereignis (6) wenigstens eine Benutzereingabe an dem Beatmungsgerät (1) und/oder an dem Diagnosegerät (2) und vorzugsweise eine zeitgleich am Beatmungsgerät (1) und Diagnosegerät (2) ausgeführte Benutzereingabe ist und wobei durch die Benutzereingabe wenigstens ein Synchronisationssignal erzeugt und dem Beatmungssignal (3) und/oder dem Diagnosesignal (4) hinzugefügt wird und wobei die Synchronisationseinheit (5) dazu geeignet und ausgebildet ist, das Synchronisationssignal als eine Signaländerung (13, 14) zu erkennen und für die Synchronisation heranzuziehen.

11. Beatmungssystem (10) nach einem der vorhergehenden Ansprüche, wobei zwischen dem Beatmungsgerät (1) und dem Diagnosegerät (2) insbesondere drahtlos Daten übertragbar sind und wobei das Ereignis (6) ein Absenden und/oder ein Eintreffen eines zu übertragenden Datenpakets ist und wobei die Synchronisationseinheit (5) dazu geeignet und ausgebildet ist, die Signalverläufe unter der Annahme zu synchronisieren, dass das Absenden und das Eintreffen des Datenpakets zeitgleich oder unter einem definierten und bei der Synchronisation berücksichtigten Zeitversatz erfolgt ist.

12. Beatmungssystem (10) nach dem vorhergehenden Anspruch, wobei die Synchronisationseinheit (5) dazu geeignet und ausgebildet ist, für die Synchronisation eine Übertragungsqualität des Datenpaktes zu berücksichtigen und insbesondere die Synchronisation zu verwerfen, falls die Übertragungsqualität unterhalb eines Schwellenwerts liegt.

13. Beatmungssystem (10) nach einem der vorhergehenden Ansprüche, wobei die Synchronisationseinheit (5) dazu geeignet und ausgebildet ist, mittels der Beatmungseinrichtung (11) die Atemgasströmung gezielt zu manipulieren, sodass im zeitlichen Verlauf des Beatmungssignals (3) und im zeitlichen Verlauf des Diagnosesignals (4) jeweils wenigstens eine wiedererkennbare Signaländerung (13, 14) auftritt, die auf einem durch die Manipulation der Atemgasströmung verursachten zeitgleichen Ereignis (6) beruhen.

14. Beatmungssystem (10) nach dem vorhergehenden Anspruch, wobei die Manipulation wenigstens eine gezielte Druckänderung und/oder Flussänderung der Atemgasströmung umfasst.

15. Beatmungssystem (10) nach einem der vorhergehenden Ansprüche, wobei die Synchronisationseinheit (5) dazu geeignet und ausgebildet ist, die synchronisierten Verläufe des Beatmungssignals (3) und des Diagnosesignals (4) auf einer gemeinsamen Zeitachse aufzutragen und insbesondere einer Anzeigeeinrichtung (25) bereitzustellen und/oder aus den synchronisierten zeitlichen Verläufen wenigstens eine Kennzahl zur Bewertung der Qualität der Beatmung zu ermitteln.

16. Beatmungssystem (10) nach einem der vorhergehenden Ansprüche, wobei die Synchronisationseinheit (5) in das Beatmungsgerät (1) integriert ist.

17. Beatmungssystem (10) nach einem der vorhergehenden Ansprüche, wobei die Sensoreinrichtung (12) des Diagnosegeräts (2) wenigstens teilweise am Körper des Patienten anordnenbar ist.

## Claims

1. A ventilation system (10) comprising at least one ventilator (1) and at least one diagnostic device (2), wherein the ventilator (1) comprises at least one ventilation apparatus (11) for generating a respiratory gas flow for ventilation and at least one detection apparatus (21) for detecting at least one ventilation signal (3) that is characteristic of the respiratory gas flow over time, and wherein the diagnostic device (2) comprises at least one sensor apparatus (12) for detecting at least one diagnostic signal (4) over time, wherein at least one synchronization unit (5) is operatively connected to the detection apparatus (21) and the sensor apparatus (12), and wherein the synchronization unit (5) is suitable and designed to examine at least one temporal waveform of the ventilation signal (3) and at least one temporal waveform of the diagnostic signal (4) in each case for at least one signal change (13, 14) caused by the same event (6) and to bring the waveform of the ventilation signal (3) and the waveform of the diagnostic signal (4) into temporal alignment such that the event (6) occurs simultaneously in both signal waveforms, wherein the ventilation signal (3) comprises a measure of a flow of the respiratory gas and/or a measure of a pressure of the respiratory gas and/or a measure of a leakage rate and the diagnostic signal (4) is taken from a group of diagnostic signal types comprising: blood gas sensor signals, ECG signals, EMG signals, inductance plethysmography signals, (body-sound) microphone signals, body position sensor signals, acceleration sensor signals, temperature sensor signals, video signals, thermal imaging signals, radar signals, ultrasound signals, wherein the synchronization unit (5) is suitable and designed to examine the temporal waveform of the ventilation signal (3) and the temporal waveform of the diagnostic signal (4) in each case for a plurality of signal changes (13, 14) which, in each case, occur in pairs and are attributable to the same event (6), and to bring the temporal waveform of the ventilation signal (3) and the temporal waveform of the diagnostic signal (4), at least in part also taking account of the further signal changes (13, 14), into temporal alignment, **characterized in that** the synchronization unit (5) is suitable and designed to detect signal changes (13, 14) that are caused by at least two different types of events and, additionally, to carry out a plausibility check as to whether the signal changes of the individual event types lead to an aligned synchronization result or not.

2. The ventilation system (10) according to the preceding claim, wherein the synchronization unit (5) is suitable and designed, by means of the signal changes (13, 14) caused by the same event (6), to detect at least one unambiguous point in time in the temporal waveform of the ventilation signal (3) and in the temporal waveform of the diagnostic signal (4) and to synchronize the signal waveforms to this point in time.

3. The ventilation system (10) according to one of the preceding claims, wherein the synchronization unit (5) is suitable and designed to determine a measure of similarity of the signal changes (13, 14) in the temporal waveform of the ventilation signal (3) and in the temporal waveform of the diagnostic signal (4), and to determine, depending on the similarity, whether the signal changes (13, 14) are attributable to the same event (6) or not.

4. The ventilation system (10) according to the preceding claim, wherein the measure of the similarity is a duration and/or an intensity and/or a symmetry property.

5. The ventilation system (10) according to the preceding claim, wherein a search is performed for signal changes occurring in pairs (13, 14) within defined time ranges.

6. The ventilation system (10) according to the preceding claim, wherein the time ranges comprise in particular a start of the therapy and an end of the therapy and at least one therapy range lying temporally therebetween.

7. The ventilation system (10) according to one of the two preceding claims, wherein the time ranges are at least 1 minute and at most 60 minutes, and are preferably 15 minutes +/- 5 minutes.

8. The ventilation system (10) according to one of the preceding claims, wherein the synchronization unit (5) is suitable and designed to detect at least one signal change (13, 14) which is attributable to an event (6) that is taken from a group of event types comprising: (acute or chronic) respiratory dysfunction (apnea, cough), movement of the patient, displaced respiratory interface (41), leakage.

9. The ventilation system (10) according to one of the preceding claims, wherein at least two types of ventilation signals that are characteristic of the respiratory gas flow are detectable by means of the detection apparatus (21) and/or wherein at least two types of diagnostic signals are detectable by means of the sensor apparatus (12), and wherein an assignment of at least one type of ventilation signal to at least one type of diagnostic signal is stored in the synchronization unit (5), and wherein the synchronization unit (5) is suitable and designed to synchronize a signal waveform (3) of a type of ventilation signal with the signal waveform (4) of a type of diagnostic signal that is associated according to the assignment.

10. The ventilation system (10) according to one of the preceding claims, wherein the event (6) is at least one user input at the ventilator (1) and/or at the diagnostic device (2) and preferably is a user input performed simultaneously at the ventilator (1) and the diagnostic device (2), and wherein at least one synchronization signal is generated by the user input and added to the ventilation signal (3) and/or the diagnostic signal (4), and wherein the synchronization unit (5) is suitable and designed to detect the synchronization signal as a signal change (13, 14) and to use it for synchronization.

11. The ventilation system (10) according to one of the preceding claims, wherein data are transmittable between the ventilator (1) and the diagnostic device (2), in particular wirelessly, and wherein the event (6) is a sending and/or a receipt of a data packet to be transmitted, and wherein the synchronization unit (5) is suitable and designed to synchronize the signal waveforms on the assumption that the sending and the receipt of the data packet take place simultaneously or with a defined time offset that is taken into account during the synchronization.

12. The ventilation system (10) according to the preceding claim, wherein the synchronization unit (5) is suitable and designed to take into account a transmission quality of the data packet for the synchronization and, in particular, to discard the synchronization if the transmission quality is below a threshold.

13. The ventilation system (10) according to one of the preceding claims, wherein the synchronization unit (5) is suitable and designed to deliberately manipulate, by means of the ventilation apparatus (11), the respiratory gas flow such that, in the temporal waveform of the ventilation signal (3) and in the temporal waveform of the diagnostic signal (4), in each case at least one identifiable signal change (13, 14) occurs that is attributable to a simultaneous event (6) caused by the manipulation of the respiratory gas flow.

14. The ventilation system (10) according to the preceding claim, wherein the manipulation comprises at least one deliberate change of the pressure and/or of the flow rate of the respiratory gas flow.

15. The ventilation system (10) according to one of the preceding claims, wherein the synchronization unit (5) is suitable and designed to plot the synchronized waveforms of the ventilation signal (3) and of the diagnostic signal (4) on a common time axis and, in particular, to provide these to a display apparatus (25) and/or to determine, from the synchronized temporal waveforms, at least one metric for evaluating the quality of the ventilation.

16. The ventilation system (10) according to one of the preceding claims, wherein the synchronization unit (5) is integrated into the ventilator (1).

17. The ventilation system (10) according to one of the preceding claims, wherein the sensor apparatus (12) of the diagnostic device (2) can be disposed at least partially on the body of the patient.

## Revendications

1. Système respiratoire (10) comprenant au moins un appareil respiratoire (1) et au moins un appareil de diagnostic (2), dans lequel l'appareil respiratoire (1) comprend au moins un dispositif respiratoire (11) destiné à générer un flux de gaz respiratoire pour une assistance respiratoire et au moins un dispositif de détection (21) destiné à détecter au moins un signal respiratoire (3) caractéristique du flux de gaz respiratoire au cours du temps, et dans lequel l'appareil de diagnostic (2) comprend au moins un dispositif capteur (12) destiné à détecter au moins un signal de diagnostic (4) au cours du temps, dans lequel au moins une unité de synchronisation (5) est en liaison fonctionnelle avec le dispositif de détection (21) et le dispositif capteur (12), et l'unité de synchronisation (5) est adaptée et configurée pour analyser au moins une évolution temporelle du signal respiratoire (3) et au moins une évolution temporelle du signal de diagnostic (4) respectivement quant à au moins une modification de signal (13, 14) provoquée par le même événement (6), et pour mettre l'évolution du signal respiratoire (3) et l'évolution du signal de diagnostic (4) en concordance temporelle de telle sorte que l'événement (6) se produit simultanément dans les deux évolutions de signal, dans lequel le signal respiratoire (3) comprend une mesure d'un flux du gaz respiratoire et/ou une mesure d'une pression du gaz respiratoire et/ou une mesure d'un taux de fuite et le signal de diagnostic (4) est choisi dans un groupe de types de signaux de diagnostic comprenant : des signaux de capteurs de gaz du sang, des signaux d'ECG, des signaux d'EMG, des signaux de pléthysmographie par induction, des signaux de microphone (sons corporels), des signaux de capteurs de position du corps, des signaux de capteurs d'accélération, des signaux de capteurs de température, des signaux vidéo, des signaux d'imagerie thermique, des signaux radar, des signaux ultrasonores, l'unité de synchronisation (5) est adaptée et configurée pour analyser, respectivement, l'évolution temporelle du signal respiratoire (3) et l'évolution temporelle du signal de diagnostic (4) quant à une pluralité de modifications de signal (13, 14) résultant, par paires, du même événement (6), et pour amener l'évolution temporelle du signal respiratoire (3) et l'évolution temporelle du signal de diagnostic (4) en concordance temporelle, au moins partiellement, en tenant également compte des autres modifications de signal (13, 14), dans lequel l'unité de synchronisation (5) est adaptée et configurée pour reconnaître des modifications de signal (13, 14) dues à au moins deux types d'événements différents et pour effectuer en outre un contrôle de plausibilité afin de vérifier si les modifications de signal de chacun des types d'événement aboutissent ou non à un résultat de synchronisation concordant.

2. Système respiratoire (10) selon la revendication précédente, dans lequel l'unité de synchronisation (5) est adaptée et configurée pour reconnaître, au moyen des modifications de signal (13, 14) provoquées par le même événement (6), au moins un moment précis dans l'évolution temporelle du signal respiratoire (3) et dans l'évolution temporelle du signal de diagnostic (4), et pour synchroniser ces évolutions sur ledit moment.

3. Système respiratoire (10) selon l'une des revendications précédentes, dans lequel l'unité de synchronisation (5) est adaptée et configurée pour déterminer une mesure de similarité des modifications de signal (13, 14) dans l'évolution temporelle du signal respiratoire (3) et dans l'évolution temporelle du signal de diagnostic (4) et, en fonction de la similarité, pour déterminer si lesdites modifications de signal (13, 14) résultent du même événement (6) ou non.

4. Système respiratoire (10) selon la revendication précédente, dans lequel la mesure de similarité est une durée et/ou une intensité et/ou une propriété de symétrie.

5. Système respiratoire (10) selon la revendication précédente, dans lequel on recherche des modifications de signal (13, 14) par paires dans des plages temporelles définies.

6. Système respiratoire (10) selon la revendication précédente, dans lequel les plages temporelles comprennent en particulier un début de thérapie et une fin de thérapie, et au moins une plage de thérapie temporelle intermédiaire.

7. Système respiratoire (10) selon l'une des deux revendications précédentes, dans lequel les plages temporelles comprennent au moins 1 minute et au maximum 60 minutes, et de préférence 15 minutes +/- 5 minutes.

8. Système respiratoire (10) selon l'une des revendications précédentes, dans lequel l'unité de synchronisation (5) est adaptée et configurée pour reconnaître au moins une modification de signal (13, 14) résultant d'un événement (6) choisi dans un groupe de types d'événement comprenant : un trouble respiratoire (aigu ou chronique) (apnée, toux), un mouvement du patient, une interface respiratoire (41) déplacée, une fuite.

9. Système respiratoire (10) selon l'une quelconque des revendications précédentes, dans lequel le dispositif de détection (21) permet de détecter au moins deux types de signaux respiratoires caractéristiques du flux de gaz respiratoire et/ou dans lequel le dispositif capteur (12) permet de détecter au moins deux types de signaux de diagnostic, et dans lequel une association d'au moins un type de signal respiratoire à au moins un type de signal de diagnostic est enregistrée dans l'unité de synchronisation (5), et dans lequel l'unité de synchronisation (5) est adaptée et configurée pour synchroniser l'évolution de signal (3) d'un type de signal respiratoire avec l'évolution de signal (4) d'un type de signal de diagnostic correspondant conformément à ladite association.

10. Système respiratoire (10) selon l'une des revendications précédentes, dans lequel l'événement (6) est au moins une entrée d'utilisateur sur l'appareil respiratoire (1) et/ou sur l'appareil de diagnostic (2) et, de préférence, une entrée d'utilisateur exécutée simultanément sur l'appareil respiratoire (1) et l'appareil de diagnostic (2), et dans lequel, par l'entrée d'utilisateur, au moins un signal de synchronisation est généré et ajouté au signal respiratoire (3) et/ou au signal de diagnostic (4), et dans lequel l'unité de synchronisation (5) est adaptée et configurée pour reconnaître le signal de synchronisation en tant que modification de signal (13, 14) et pour l'utiliser pour la synchronisation.

11. Système respiratoire (10) selon l'une des revendications précédentes, dans lequel des données sont transmissibles, notamment sans fil, entre l'appareil respiratoire (1) et l'appareil de diagnostic (2), et dans lequel l'événement (6) est un envoi et/ou une réception d'un paquet de données à transmettre, et dans lequel l'unité de synchronisation (5) est adaptée et configurée pour synchroniser les évolutions des signaux en supposant que l'envoi et la réception du paquet de données ont eu lieu simultanément ou avec un décalage temporel défini et pris en compte lors de la synchronisation.

12. Système respiratoire (10) selon la revendication précédente, dans lequel l'unité de synchronisation (5) est adaptée et configurée pour prendre en compte, aux fins de la synchronisation, une qualité de transmission du paquet de données et, en particulier, pour rejeter la synchronisation si la qualité de transmission est inférieure à une valeur seuil.

13. Système respiratoire (10) selon l'une des revendications précédentes, dans lequel l'unité de synchronisation (5) est adaptée et configurée pour manipuler de manière ciblée le flux de gaz respiratoire au moyen du dispositif respiratoire (11), de sorte qu'au moins une modification de signal (13, 14) reconnaissable, résultant d'un événement (6) simultané provoqué par la manipulation du flux de gaz respiratoire, apparaît respectivement dans l'évolution temporelle du signal respiratoire (3) et dans l'évolution temporelle du signal de diagnostic (4).

14. Système respiratoire (10) selon la revendication précédente, dans lequel la manipulation comprend au moins une modification ciblée de la pression et/ou du flux du gaz respiratoire.

15. Système respiratoire (10) selon l'une des revendications précédentes, dans lequel l'unité de synchronisation (5) est adaptée et configurée pour représenter les évolutions synchronisées du signal respiratoire (3) et du signal de diagnostic (4) sur un axe temporel commun et notamment pour les fournir à un dispositif d'affichage (25) et/ou pour déterminer, à partir des évolutions temporelles synchronisées, au moins un indicateur pour l'évaluation de la qualité de l'assistance respiratoire.

16. Système respiratoire (10) selon l'une des revendications précédentes, dans lequel l'unité de synchronisation (5) est intégrée dans l'appareil respiratoire (1).

17. Système respiratoire (10) selon l'une des revendications précédentes, dans lequel le dispositif capteur (12) de l'appareil de diagnostic (2) est susceptible d'être disposé au moins partiellement sur le corps du patient.
